# EUROPEAN PATENT APPLICATION

(11) **EP 4 495 244 A1**
(43) Date of publication of application: **22.01.2025**
(21) Application number: 23770896.1
(22) Date of filing: 16.03.2023
(51) Int. Cl.: C12N 15/55, C12N 1/15, C12N 1/19, C12N 1/21, C12N 5/10, C12N 9/80, C12N 15/63, C12P 21/02

(54) **MODIFIED PROTEIN GLUTAMINASE**

(30) Priority: 16.03.2022 JP 2022041629; 24.05.2022 JP 2022084393
(71) Applicant: Amano Enzyme Inc., Nagoya-shi Aichi 460-8630 (JP)
(72) Inventor: ONO, Atsushi, Kakamigahara-shi, Gifu 509-0109 (JP); YOSHIDA, Kazunori, Kakamigahara-shi, Gifu 509-0109 (JP); ARIYOSHI, Ryutaro, Kakamigahara-shi, Gifu 509-0109 (JP)
(74) Representative: Müller-Boré & Partner Patentanwälte PartG mbB
(86) International application number: PCT/JP2023/010448
(87) International publication number: WO 2023/176943

(57) **Abstract**

The purpose of the present invention is to provide a protein glutaminase having improved oxidation stability. A protein glutaminase including a polypeptide including an amino acid sequence in which at least one substitution among (A) substitution of a valine residue or an alanine residue for the position 121 amino acid residue and (B) substitution of a cysteine residue or an aspartic acid residue for the position 142 amino acid residue has been introduced in an amino acid sequence shown in SEQ ID NO: 1 improves the oxidation stability over that of a polypeptide including the amino acid sequence shown in SEQ ID NO: 1.

## Description

### Technical Field

The present invention relates to a modified protein glutaminase. More specifically, the present invention relates to a protein glutaminase modified so as to improve oxidation stability.

### Background Art

Protein glutaminase is an enzyme that acts on protein being a polymer, and catalyzes a reaction in which an amide group-containing side chain is decomposed (i.e., deamidated) without cleavage of peptide bonds and crosslinking of the protein. Protein glutaminase deamidates glutamine residues in protein to generate negatively charged carboxyl groups, so that the protein undergoes various characteristic changes. For example, an increase in hydration force and a rise in electrostatic repulsion due to a decrease in isoelectric point of protein leads to a decrease in interaction between proteins (i.e., a decrease in association property), and thus enhances the solubility and water dispersibility of the protein. In addition, the exposure of inner hydrophobic regions due to a change in higher order structure of protein imparts interfacial activity to the protein, and thus improves the emulsifying capacity, emulsion stability, foaming property, and foam stability of the protein. Protein glutaminase can significantly change the properties of protein as described above, and therefore have dramatically expanded the uses of protein. For this reason, protein glutaminase is very useful, and has attracted much attention in the art.

Protein glutaminase was first discovered in Chryseobacterium proteolyticum in 2000 (Non Patent Literature 1). Since then, protein glutaminase derived from C. proteolyticum has been industrially used as the only active ingredient of protein glutaminase enzyme preparation for a long time.

### Citation List

### Non Patent Literature

NPL 1: A novel protein-deamidating enzyme from Chryseobacterium proteolyticum sp. nov., a newly isolated bacterium from soil. Applied and Environmental Microbiology 2000; 66(8): 3337-43

### Summary of Invention

### Technical Problem

Protein glutaminase derived from C. proteolyticum is poor in oxidation stability and difficult to preserve for a long period of time. Therefore, in the present situation, handling of protein glutaminase derived from C. proteolyticum requires use or storage in an environment where oxidation is unlikely to occur.

On the other hand, in view of the possibility of further expansion of the uses of protein glutaminase, which is expected from its high usefulness, it is desirable that protein glutaminase be capable of being stored for a long period of time, and for that purpose, improvement of the oxidation stability of protein glutaminase is desired.

Accordingly, an object of the present invention is to provide a protein glutaminase having improved oxidation stability.

### Solution to Problem

The present inventors have extensively conducted studies, and resultantly found a new mutation that can improve oxidation stability of the protein glutaminase (specifically, improve the remaining activity of protein glutaminase after hydrogen peroxide treatment as compared to the remaining activity of wild-type protein glutaminase). The present invention has been completed on the basis of these findings. That is, the present invention provides inventions of aspects as listed below.

Item 1. A modified protein glutaminase including one of the following polypeptides (I) to (III):
   (I) a polypeptide consisting of an amino acid sequence obtained by introducing at least one of (A) a substitution of the amino acid residue at position 121 with a valine residue or an alanine residue, (B) a substitution of the amino acid residue at position 142 with a cysteine residue or an aspartic acid residue, (C) a substitution of the amino acid residue at position 22 with a tyrosine residue, (D) a substitution of the amino acid residue at position 26 with a tyrosine residue or a cysteine residue, (E) a substitution of the amino acid residue at position 30 with a threonine residue, (F) a substitution of the amino acid residue at position 33 with a valine residue, (G) a substitution of the amino acid residue at position 35 with a serine residue, (H) a substitution of the amino acid residue at position 36 with an isoleucine residue, (I) a substitution of the amino acid residue at position 43 with a phenylalanine residue, (J) a substitution of the amino acid residue at position 73 with an isoleucine residue, (K) a substitution of the amino acid residue at position 113 with a lysine residue, (L) a substitution of the amino acid residue at position 156 with a proline residue, (M) a substitution of the amino acid residue at position 157 with a phenylalanine residue, (N) a substitution of the amino acid residue at position 169 with a phenylalanine residue, (O) a substitution of the amino acid residue at position 170 with a threonine residue, (P) a substitution of the amino acid residue at position 182 with an arginine residue, and (Q) a substitution of the amino acid residue at position 183 with a glycine residue, into the amino acid residue set forth as SEQ ID NO: 1;
   (II) a polypeptide in which one or several amino acid residues other than the substituted amino acid residues are substituted, added, inserted or deleted in the amino acid sequence in which at least one of the substitutions (A) to (Q) is introduced, and the remaining activity of protein glutaminase after hydrogen peroxide treatment is improved as compared to the remaining activity of the polypeptide consisting of the amino acid sequence set forth as SEQ ID NO: 1; and
   (III) a polypeptide in which the sequence identity of regions that do not include the substituted amino acid sequences is 70% or more in the amino acid sequence in which at least one of the substitutions (A) to (Q) is introduced, and the remaining activity of protein glutaminase after hydrogen peroxide treatment is improved as compared to the remaining activity of the polypeptide consisting of the amino acid sequence set forth as SEQ ID NO: 1.
Item 2. A DNA encoding the modified protein glutaminase according to item 1.
Item 3. An expression cassette or a recombinant vector including the DNA according to item 2.
Item 4. A transformant obtained by transforming a host using the expression cassette or recombinant vector according to item 3.
Item 5. A method for producing a modified protein glutaminase, including the step of culturing the transformant according to item 4.
Item 6. An enzyme agent including the modified protein glutaminase according to item 1.
Item 7. A modifier for a protein material, including the modified protein glutaminase according to item 1.
Item 8. A method for producing a modified protein material, including the step of applying the modified protein glutaminase according to item 1 to a protein material. Advantageous Effect of Invention

According to the present invention, a modified protein glutaminase having improved oxidation stability is provided.

### Description of Embodiments

Hereinafter, the present invention will be described in detail. The 20 types of amino acid residues in the amino acid sequence may be represented by one character in abbreviation. Specifically, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In the amino acid sequence described herein, the left end is a N-terminus, and the right end is a C-terminus.

The term "non-polar amino acid", as used herein, includes glycine, alanine, valine, leucine, isoleucine, proline, methionine, phenylalanine, and tryptophan. The term "non-charged amino acids", as used herein, includes glycine, serine, threonine, cysteine, tyrosine, asparagine, and glutamine. The "acidic amino acid" includes aspartic acid and glutamic acid. The "basic amino acid" includes lysine, arginine, and histidine.

The term "substitution", as used herein, includes not only a case where a substitution of an amino acid residue is artificially introduced, but also a case where a substitution of an amino acid residue is naturally introduced, that is, a case where amino acid residues are intrinsically different. The substitution of an amino acid residue, as used herein, may be an artificial substitution or a natural substitution, but is preferably an artificial substitution.

### 1. Modified protein glutaminase

The modified protein glutaminase of the present invention includes one of the following polypeptides (I) to (III).

(I) A polypeptide consisting of an amino acid sequence obtained by introducing at least one of
   (A) a substitution of the amino acid residue at position 121 with a valine residue or an alanine residue,
   (B) a substitution of the amino acid residue at position 142 with a cysteine residue or an aspartic acid residue,
   (C) a substitution of the amino acid residue at position 22 with a tyrosine residue,
   (D) a substitution of the amino acid residue at position 26 with a tyrosine residue or a cysteine residue,
   (E) a substitution of the amino acid residue at position 30 with a threonine residue,
   (F) a substitution of the amino acid residue at position 33 with a valine residue,
   (G) a substitution of the amino acid residue at position 35 with a serine residue,
   (H) a substitution of the amino acid residue at position 36 with an isoleucine residue,
   (I) a substitution of the amino acid residue at position 43 with a phenylalanine residue,
   (J) a substitution of the amino acid residue at position 73 with an isoleucine residue,
   (K) a substitution of the amino acid residue at position 113 with a lysine residue,
   (L) a substitution of the amino acid residue at position 156 with a proline residue,
   (M) a substitution of the amino acid residue at position 157 with a phenylalanine residue,
   (N) a substitution of the amino acid residue at position 169 with a phenylalanine residue,
   (O) a substitution of the amino acid residue at position 170 with a threonine residue,
   (P) a substitution of the amino acid residue at position 182 with an arginine residue, and
   (Q) a substitution of the amino acid residue at position 183 with a glycine residue,
      into the amino acid sequence set forth as SEQ ID NO: 1.
(II) a polypeptide in which one or several amino acid residues other than the substituted amino acid residues are substituted, added, inserted or deleted in the amino acid sequence in which at least one of the substitutions (A) to (Q) is introduced, and the remaining activity of protein glutaminase after hydrogen peroxide treatment is improved as compared to the remaining activity of the polypeptide consisting of the amino acid sequence set forth as SEQ ID NO: 1.
(III) a polypeptide in which the sequence identity of regions that do not include the substituted amino acid sequences is 70% or more in the amino acid sequence in which at least one of the substitutions (A) to (Q) is introduced, and the remaining activity of protein glutaminase after hydrogen peroxide treatment is improved as compared to the remaining activity of the polypeptide consisting of the amino acid sequence set forth as SEQ ID NO: 1.

The amino acid sequence set forth as SEQ ID NO: 1 is a mature sequence from the full-length sequence (sequence including signal sequence and pro-sequence) of protein glutaminase derived from Chryseobacterium proteolyticum, which is set forth as SEQ ID NO: 2.

The polypeptides (I) to (III) may be polypeptides containing the substitutions (A) to (Q) singly (single-substituted forms of protein glutaminase derived from C. proteolyticum), or may be polypeptides containing the substitutions (A) to (Q) in combination of two or more thereof (multi-substituted forms of protein glutaminase derived from C. proteolyticum). Examples of preferred polypeptides, among the polypeptides (I) to (III), include: polypeptides containing at least one of substitutions (A), (B), (D), (F), (G), (H), (J), (L), (M), (N) and (P) among the substitutions (A) to (Q); polypeptides containing substitutions (E) and (K) among the substitutions (A) to (Q); polypeptides containing substitutions (D), (I) and (Q) among the substitutions (A) to (Q); and polypeptides containing substitutions (C) and (O) among the substitutions (A) to (Q).

As a specific example of the polypeptide (I), the specific amino acid sequences of the polypeptides having, as the substitution (A), a substitution with a valine residue and a substitution with an alanine residue are set forth as SEQ ID NO: 3 and SEQ ID NO: 4, respectively, and the specific amino acid sequences of the polypeptides having, as the substitution (B), a substitution with a cysteine residue and a substitution with an aspartic acid residue are set forth as SEQ ID NO: 5 and SEQ ID NO: 6, respectively.

In the polypeptide (II), the amino acid modification introduced may include, among substitution, addition, insertion, and deletion, only one type of modification (for example, only substitution) or two or more types of modifications (for example, substitution and insertion). In the polypeptide (II), the number of amino acid differences at an arbitrary difference site may be 1 or several number, and is, for example, 1 to 18, preferably 1 to 10, more preferably 1 to 8, 1 to 7, 1 to 6, 1 to 5, or 1 to 4, still more preferably 1 to 3, and particularly preferably 1 or 2, or 1.

In the polypeptide (III), the sequence identity with the amino acid sequence set forth as SEQ ID NO: 1 may be 70% or more, and is preferably 80% or more, or 85% or more, more preferably 90% or more, or 93% or more, still more preferably 95% or more, even more preferably 98% or more, furthermore preferably 98.5% or more, or 99% or more, and particularly preferably 99.3% or more, or 99.5% or more.

Here, in the polypeptide (III), the sequence identity with each amino acid sequence set forth as SEQ ID NO: 1 is sequence identity calculated by comparison with the amino acid sequence set forth as SEQ ID NO: 1. The "sequence identity" indicates a value of identity of an amino acid sequence obtained by the bl2seq program (Tatiana A. Tatsusova, Thomas L. Madden, FEMS Microbiol. Lett., Vol. 174, p247-250, 1999) of BLASTPACKAGE [sgi32 bit edition, Version 2.0.12; available from National Center for Biotechnology Information (NCBI)]. The parameters may be set to Gap insertion Cost value: 11 and Gap extension Cost value: 1.

In the polypeptides of (II) and (III), amino acid residues corresponding to position 42 (cysteine residue), position 83 (histidine residue) and position 103 (aspartic acid residue) in the amino acid sequence set forth as SEQ ID NO: 1 may be active catalyst residues, and therefore it is desirable not to introduce substitutions or deletions at these sites.

When an amino acid substitution is introduced to SEQ ID NO: 1 in the polypeptides (II) and (III), a preferred type of the amino acid substitution introduced includes conservative substitution. That is, for example, the substitution in the polypeptides (II) and (III) is such that when the amino acid before substitution is a non-polar amino acid, a substitution with another non-polar amino acid is introduced, or when the amino acid before substitution is a non-charged amino acid, a substitution with another non-charged amino acid is introduced, or when the amino acid before substitution is an acidic amino acid, a substitution with another acidic-polar amino acid is introduced, or when the amino acid before substitution is a basic amino acid, a substitution with another basic amino acid is introduced.

Specific examples of the polypeptides (II) and (III) include polypeptides obtained by introducing the substitution into protein glutaminase analogous to protein glutaminase derived from C. proteolyticum. Examples of the analogous protein glutaminase into which the substitution is introduced include protein glutaminase derived from Chryseobacterium sp., more specifically an amino acid sequence set forth as SEQ ID NO: 7 or SEQ ID NO: 8, which is a mature sequence of protein glutaminase derived from Chryseobacterium sp., or an amino acid sequence of SEQ ID NO: 7 or SEQ ID NO: 8 in which the amino acid residue at position 115 is substituted with another amino acid residue (for example, a serine residue). SEQ ID NO: 7 has a sequence identity of 86.9% with SEQ ID NO: 1 and SEQ ID NO: 8 has a sequence identity of 87.4% with SEQ ID NO: 1. The full-length sequence of SEQ ID NO: 7 is the amino acid sequence set forth as SEQ ID NO: 9, and the full-length sequence of SEQ ID NO: 8 is the amino acid sequence set forth as SEQ ID NO: 10.

The polypeptides (I) to (III) above have protein glutaminase activity, and the property that oxidation stability is improved. The term "property that oxidation stability is improved" means that the remaining activity of protein glutaminase after hydrogen peroxide treatment is improved as compared to the remaining activity of a polypeptide consisting of the amino acid sequence set forth as SEQ ID NO: 1 (wild-type protein glutaminase derived from Chryseobacterium proteolyticum), specifically, it is mentioned that the remaining activity when 0.1 mg/mL of aqueous polypeptide solution is treated with hydrogen peroxide at a final concentration of 0.001% at 37°C for 1 hour for 0.1 mg/mL (hereinafter, also referred to as "post-oxidation treatment remaining activity A_{0.001%}") is 2 times or more of the post-oxidation treatment remaining activity A_{0.001%} of wild-type protein glutaminase. As a preferred example of the oxidation stability of the polypeptides (I) to (III) above, the post-oxidation treatment remaining activity A_{0.001%} is 3 times or more, more preferably 4 times or more, still more preferably 5 times or more, and even more preferably 5.5 times or more of the post-oxidation treatment remaining activity A_{0.001%} of wild-type protein glutaminase. The upper limit of the post-oxidation treatment remaining activity A_{0.001%} of the polypeptides (I) to (III) is not limited, and is, for example, 20 times or less, or 10 times or less of the post-oxidation treatment remaining activity A_{0.001%} of wild-type protein glutaminase. When presented in terms of the remaining activity when 0.1 mg/mL of the aqueous polypeptide solution is treated with hydrogen peroxide at a final concentration of 0.0025% at 37°C for 1 hour (hereinafter, also referred to as "post-oxidation treatment remaining activity B_{0.0025%}"), the property that oxidation stability is improved in the polypeptides (I) to (III) is, for example, 5 times or more, preferably 7 times or more, still more preferably 10 times or more, and even more preferably 20 times or more of the post-oxidation treatment remaining activity B_{0.0025%} of wild-type protein glutaminase. The upper limit of the post-oxidation treatment remaining activity B_{0.025%} of the polypeptides (I) to (III) is not limited, and is, for example, 60 times or less, or 50 times or less of the post-oxidation treatment remaining activity B_{0.0025%} of wild-type protein glutaminase.

The specific activity of the polypeptides (I) to (III) is not limited, and the specific activity of the polypeptides (I) to (III) at 37°C is, for example, 20% or more, 40% or more, or 60% or more when the specific activity of the polypeptide consisting of the amino acid sequence set forth as SEQ ID NO: 1 (wild-type protein glutaminase derived from Chryseobacterium proteolyticum) at 37°C is defined as 100%. The specific activity of the polypeptides (I) to (III) is preferably equivalent to the specific activity of the polypeptide consisting of the amino acid sequence set forth as SEQ ID NO: 1 (wild-type protein glutaminase derived from Chryseobacterium proteolyticum). The equivalent specific activity, specifically the specific activity of the polypeptides (I) to (III) at 37°C when the specific activity, at 37°C, of the polypeptide consisting of the amino acid sequence set forth as SEQ ID NO: 1 is defined as 100% is 80% or more, preferably 90% or more, more preferably 95% or more, still more preferably 100% or more, and even more preferably 105% or more.

The amount of an enzyme producing ammonia at 1 µmol per minute using Z-Gln-Gly (benzyloxycarbonyl-L-glutaminylglycine) as a substrate is defined as one unit (1 U) of the protein glutaminase activity.

The modified protein glutaminase of the present invention may be used as an active ingredient of an enzyme agent described later, or may be used for forming a part of a larger protein integrated in the form of a fusion protein or the like with peptides or proteins consisting of other amino acid sequences (hereinafter, also referred to as "other proteins etc."). Examples of the other proteins etc. include peptides used for purification of proteins, such as a polyhistidine residue, and derived from an additional sequence for securing stability of mRNA during recombinant production.

### 2. DNA

The DNA of the present invention is DNA encoding the modified protein glutaminase described in "1. Modified protein glutaminase" above.

The DNA of the present invention is not limited as long as it has a nucleotide sequence encoding a modified protein glutaminase including any of the polypeptides (I) to (III) described in "1. Modified protein glutaminase" above. Examples of the nucleotide sequence of DNA encoding the amino acid sequence set forth as SEQ ID NO: 1 (protein glutaminase derived from Chryseobacterium proteolyticum), which is a reference sequence for the polypeptides (I) to (III), include SEQ ID NO: 11. Therefore, those skilled in the art can appropriately design the DNA of the present invention using SEQ ID NO: 11 as a reference sequence.

Examples of the DNA of the present invention include the following DNAs [i] to [iii].

[i] A DNA consisting of a nucleotide sequence obtained by introducing at least one of
   (a) a substitution at positions 361 to 363 with a nucleotide sequence encoding a valine residue or an alanine residue,
   (b) a substitution at positions 424 to 426 with a nucleotide sequence encoding a cysteine residue or an aspartic acid residue,
   (c) a substitution at positions 64 to 66 with a nucleotide sequence encoding a tyrosine residue,
   (d) a substitution at positions 76 to 78 with a nucleotide sequence encoding a tyrosine residue or a cysteine residue,
   (e) a substitution at positions 88 to 90 with a nucleotide sequence encoding a threonine residue,
   (f) a substitution at positions 97 to 99 with a nucleotide sequence encoding a valine residue,
   (g) a substitution at positions 103 to 105 with a nucleotide sequence encoding a serine residue,
   (h) a substitution at positions 106 to 108 with a nucleotide sequence encoding an isoleucine residue,
   (i) a substitution at positions 127 to 129 with a nucleotide sequence encoding a phenylalanine residue,
   (j) a substitution at positions 217 to 219 with a nucleotide sequence encoding an isoleucine residue,
   (k) a substitution at positions 337 to 339 with a nucleotide sequence encoding a lysine residue,
   (l) a substitution at positions 466 to 468 with a nucleotide sequence encoding a proline residue,
   (m) a substitution at positions 469 to 471 with a nucleotide sequence encoding a phenylalanine residue,
   (n) a substitution at positions 505 to 507 with a nucleotide sequence encoding a phenylalanine residue,
   (o) a substitution at positions 508 to 510 with a nucleotide sequence encoding a threonine residue,
   (p) a substitution at positions 544 to 546 with a nucleotide sequence encoding an arginine residue, and
   (q) a substitution at positions 547 to 549 with a nucleotide sequence encoding a glycine residue,
      into the nucleotide sequence set forth as SEQ ID NO: 11.
[ii] A DNA encoding a polypeptide in which the remaining activity of protein glutaminase after hydrogen peroxide treatment is improved as compared to the remaining activity of the polypeptide consisting of the amino acid sequence set forth as SEQ ID NO: 1, the DNA hybridizing under stringent conditions with DNA consisting of a nucleotide sequence complementary to the DNA [i].
[iii] A DNA encoding a polypeptide in which the remaining activity of protein glutaminase after hydrogen peroxide treatment is improved as compared to the remaining activity of the polypeptide consisting of the amino acid sequence set forth as SEQ ID NO: 1, the DNA having a homology of 70% or more with the DNA [i].

As a specific example of the DNA [i], the specific nucleotide sequences of DNAs having, as the substitution [a], a substitution with a nucleotide sequence encoding a valine residue and a substitution with a nucleotide sequence encoding an alanine residue are set forth as SEQ ID NO: 12 and SEQ ID NO: 13, respectively, and the specific nucleotide sequences of the DNAs having, as the substitution [b], a substitution with a nucleotide sequence encoding a cysteine residue and a substitution with a nucleotide sequence encoding an aspartic acid residue are set forth as SEQ ID NO: 14 and SEQ ID NO: 15, respectively.

For the DNA [ii], the term "under stringent conditions" refers to conditions in which DNA is held at 50°C to 65°C for 4 hours to one night in 6 × SSC (1 × SSC consists of 0.15 M NaCl and 0.015 M sodium citrate at a pH of 7.0) containing 0.5% SDS, 5 × Denhartz's [0.1% bovine serum albumin (BSA), 0.1% polyvinyl pyrrolidone, 0.1% Ficoll 400] and 100 µg/ml of salmon sperm DNA.

The hybridization under stringent conditions is performed specifically by the following method. That is, a nylon membrane on which a DNA library or a cDNA library is immobilized is prepared, and the nylon membrane is blocked at 65°C in a prehybridization solution containing 6 × SSC, 0.5% SDS, 5 × Denhartz's, and 100 µg/ml of salmon sperm DNA. Thereafter, each probe labeled with ³²P is added to the nylon membrane, which is held overnight at 65°C. The nylon membrane is washed at room temperature for 10 minutes in 6 × SSC at room temperature for 10 minutes in 2 × SSC containing 0.1% SDS, and at 45°C for 30 minutes in 0.2 × SSC containing 0.1% SDS, and then subjected to autoradiography, whereby DNA hybridized specifically with the probe can be detected.

For the DNA [iii], the homology may be 70% or more, and is preferably 80% or more, or 85% or more, more preferably 90% or more, or 93% or more, still more preferably 95% or more, even more preferably 98% or more, furthermore preferably 98.5% or more, or 99% or more, and particularly preferably 99.3% or more, or 99.5% or more.

Here, the "homology" of DNA is calculated using published or marketed software with an algorithm that performs comparison using a reference sequence as a query sequence. Specifically, BLAST, FASTA, GENETYX (manufactured by GENETYX K.K.), or the like can be used, and they may be used by being set as default parameters.

The DNA of the present invention can be obtained by, for example, introducing at least one of the substitutions (a) to (q) into DNA encoding a polypeptide consisting of the amino acid sequence set forth as SEQ ID NO: 1 (that is, protein glutaminase derived from C. proteolyticum) or its analogous protein glutaminase (as described above, specific examples include Chryseobacterium sp, more specifically a polypeptide consisting of the amino acid sequence set forth as SEQ ID NO: 7 or SEQ ID NO: 8, or an amino acid sequence of SEQ ID NO: 7 or SEQ ID NO: 8 in which the amino acid residue at position 115 is substituted with another amino acid residue (for example, a serine residue)). In addition, the DNA of the present invention can be artificially synthesized by a method of total synthesis of genes.

In the case of DNA encoding a polypeptide consisting of the amino acid sequence set forth as SEQ ID NO: 1, from a nucleic acid construct such as a plasmid in which the nucleotide sequence set forth as SEQ ID NO: 11 is incorporated, the nucleotide sequence can be acquired by a conventional method based on PCR. Together with the nucleotide sequence set forth as SEQ ID NO: 11, a sequence obtained by adding a signal sequence and pro-sequence to the 5'-terminus side of the nucleotide sequence can be incorporated into the nucleic acid construct. Examples of the sequence to which such a signal sequence and a pro-sequence are added include the nucleotide sequence set forth as SEQ ID NO: 16 and encoding the full-length sequence of protein glutaminase derived from C. proteolyticum, which is set forth as SEQ ID NO: 2, and the nucleotide sequence set forth as SEQ ID NO: 17 or SEQ ID NO: 18 and encoding the full-length sequence of protein glutaminase derived from Chryseobacterium sp. which is analogous protein glutaminase, which is set forth as SEQ ID NO: 9 or SEQ ID NO: 10, respectively.

As the method for artificially modifying an amino acid sequence by introducing a mutation into a gene, known methods such as a Kunkel method and a Gapped duplex method, and a mutation introduction kit using a site-directed mutagenesis method, for example, QuikChange (trademark) Site-Directed Mutagenesis Kit (Stratagene Company), GeneTailor (trademark) Site-Directed Mutagenesis System (Invitrogen Company), TaKaRa Site-Directed Mutagenesis System (Mutan-K, Mutan-Super Express Km, etc.: Takara Bio Inc.), and the like can be used.

The DNA of the present invention includes various kinds of DNA derived from codon degeneracy. Artificial production of various kinds of DNA encoding the same amino acid sequence can be easily performed using a known genetic engineering method. For example, in genetic engineering production of protein, the expression level of a protein of interest may be low in the case where the frequency of use of a codon used on an original gene encoding the protein is low in the host. In this a case, high expression of the protein of interest can be achieved by optimizing the frequency of codon usage for the host without changing the encoded amino acid sequence.

As an index of the frequency of codon usage, the total optimum frequency of codon usage for the host may be adopted for each codon. The optimal codon is defined as a codon, the usage frequency of which is the highest among codons corresponding to the same amino acid. The frequency of codon usage is not limited as long as it is optimized for the host, and examples of the optimal codon of E. coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), G: glycine (ggc).

The nucleotide sequence of DNA in which a mutation is introduced into a base sequence can be confirmed by sequencing by a conventional method. Specific examples of the sequencing method include a dideoxynucleotide chain termination method (Sanger et al. (1977) Proc. Natl. Acad. Sci. USA 74: 5463), and a sequence analysis method using an appropriate DNA sequencer. Examples of the method for confirming whether DNA encodes a polypeptide of interest include a method in which a sequenced nucleotide sequence is compared with an unsubstituted nucleotide sequence such as the nucleotide sequence set forth as SEQ ID NO: 11, and a method in which an amino acid sequence deduced from the sequenced nucleotide sequence is compared with an unsubstituted amino acid sequence such as the amino acid sequence set forth as SEQ ID NO: 1.

### 3. Expression cassette or recombinant vector

The expression cassette or recombinant vector of the present invention contains the DNA of the present invention which is described in "2. DNA" above. The expression cassette or recombinant vector of the present invention can be obtained by connecting a promoter and a terminator to the DNA of the present invention, or inserting the expression cassette of the present invention or the DNA of the present invention into the expression vector.

The expression cassette of the present invention or the recombinant vector of the present invention may contain, as a control factor, transcription elements such as an enhancer, a CCAAT box, a TATA box, or an SPI site, if necessary, in addition to the promoter and the terminator. These control factors may be operably connected to the DNA of the present invention. The term "operably connected" means that the DNA of the present invention and various control factors that regulate the DNA of the present invention are connected in a state of being operable in host cells.

As for the recombinant vector of the present invention, an expression vector for genetic recombination, which is constructed from a phage, a plasmid, or a virus capable of autonomously growing in a host, is suitable. Such an expression vector is known, and examples thereof include commercially available expression vectors such as pQE-based vectors (Qiagen Corporation), pDR540 and pRIT2T (GE Healthcare Bio-Sciences AB), and pET-based vectors (Merck KGaA). For the expression vector, an appropriate combination with host cells may be selected and used. For example, when E. coli is used as host cells, a combination of a pET-based vector and a DH5α E. coli strain, a combination of a pET-based vector and a BL21 (DE3) E. coli strain, or a combination of a pDR540 vector and a JM109 E. coli strain may be used.

### 4. Transformant

The transformant of the present invention is obtained by transforming a host with the expression cassette or recombinant vector of the present invention described in "3. Expression cassette or recombinant vector" above.

The host for use in production of the transformant of the present invention is not limited as long as it can undergo introduction of a gene, ensures the stability of the expression cassette or recombinant vector, and is capable of autonomously growing and expressing the trait of a gene containing the DNA of the present invention. Preferred examples thereof include bacteria belonging to the genus Escherichia such as Escherichia coli, the genus Bacillus such as Bacillus subtilis, the genus Pseudomonas such as Pseudomonasputida, and the genus Chryseobacterium proteolyticum such as Chryseobacterium proteolyticum; and yeast, and may also be animal cells, insect cells, plant cells, and the like.

The transformant of the present invention can be obtained by introducing the expression cassette of the present invention or the recombinant vector of the present invention into a host. The place where the DNA of the present invention is introduced is not limited as long as a gene of interest can be expressed, and the DNA may be introduced onto a plasmid or a genome. Examples of the specific method for introducing the expression cassette of the present invention or the recombinant vector of the present invention include a recombinant vector method and a genome edition method. Conditions for introducing the expression cassette or recombinant vector into the host may be appropriately set according to the type of the host and the like. When the host is bacteria, for example, a method using competent cells under calcium ion treatment and an electroporation method are applicable. When the host is yeast, for example, an electroporation method, a spheroplast method, and a lithium acetate method are applicable. When the host is animal cells, for example, an electroporation method, a calcium phosphate method, and a lipofection method are applicable. When the host is insect cells, for example thereof, a calcium phosphate method, a lipofection method, and an electroporation method are applicable. When the host is plant cells, for example, an electroporation method, an agrobacterium method, a particle gun method, and a PEG method are applicable.

Whether the expression cassette of the present invention or the recombinant vector of the present invention has been incorporated into a host can be confirmed by a PCR method, a Southern hybridization method, a Northern hybridization method, or the like.

When whether the expression cassette of the present invention or the recombinant vector of the present invention has been incorporated into a host is confirmed by a PCR method, for example, genomic DNA, the expression cassette, or the recombinant vector may be separated and purified from a transformant.

For example, when the host is bacteria, the expression cassette or recombinant vector is separated and purified with a lysate obtained by lysing bacteria. As a method of lysis, for example, treatment with a lytic enzyme such as lysozyme is performed, and if necessary, a protease, other enzymes, and a surfactant such as sodium lauryl sulfate (SDS) are used in combination.

Physical crushing methods such as freeze-thaw and French press treatment may also be combined. The DNA can be separated and purified from the lysate by appropriately combining, for example, deproteinization treatment based on phenol treatment and protease treatment, ribonuclease treatment, alcohol precipitation treatment, and a commercially available kit.

DNA can be cleaved by, for example, restriction enzyme treatment under a conventional method. As the restriction enzyme, for example, a type II restriction enzyme that acts on a specific nucleotide sequence is used. The DNA and the expression cassette or expression vector are bound to each other using, for example, a DNA ligase.

Thereafter, a primer specific to the DNA of the present invention is designed using the separated and purified DNA as a template, and PCR is performed. The amplification product obtained by PCR is subjected to agarose gel electrophoresis, polyacrylamide gel electrophoresis, capillary electrophoresis, or the like, and stained with ethidium bromide, SYBR Green solution and the like. This enables the amplification product to be detected as a band, thereby confirming that transformation has occurred.

It is also possible to detect the amplification product by performing PCR using a primer labeled with a fluorescent dye or the like in advance. A method may also be utilized in which the amplification product is bound to a solid phase such as a microplate and confirm the amplification product by fluorescence, an enzymatic reaction, or the like.

### 5. Method for producing modified protein glutaminase

The method for producing a modified protein glutaminase according to the present invention is a method for producing an enzyme described "1. Modified protein glutaminase" above, the method including the step of culturing the transformant of the present invention. When at least one of the substitutions (A) to (Q) contained in the modified protein glutaminase is naturally introduced, the modified protein glutaminase can be obtained by a production method including the step of culturing microorganisms that produce the modified protein glutaminase.

The conditions for the culture may be appropriately set according to the nutritional and physiological properties of the transformant or the microorganisms, and liquid culture is preferable. In the case of industrial production, aerated and stirred culture is preferable. As nutrient sources of the medium, those required for growth of the transformant or the microorganisms can be used. The carbon source may be any consumable carbon compound, and examples thereof include glucose, sucrose, lactose, maltose, molasses, and pyruvic acid. The nitrogen source may be any consumable nitrogen compound, and examples thereof include peptone, meat extract, yeast extract, casein hydrolysate, and soybean cake alkaline extract. In addition to the carbon source and the nitrogen source, for example, phosphates, carbonates, sulfates, salts of magnesium, calcium, potassium, iron, manganese, zinc and the like, specific amino acids, specific vitamins, and the like may be used if necessary.

The culture temperature can be appropriately set as long as the transformant of the present invention or the microorganisms can grow, and the transformant or the microorganisms produce a modified protein glutaminase. The culture temperature is preferably about 15 to 37°C. The culture may be completed at an appropriate time that is judged to be a time when the modified protein glutaminase reaches the highest yield, and the culture time is usually about 12 to 48 hours.

After the transformant or the microorganisms are cultured, a method such as centrifugation is applied to the culture solution to collect the culture supernatant and/or bacterial cells. A mechanical method such as ultrasonication or French press or treatment with a lytic enzyme such as lysozyme is applied to the bacterial cells, and if necessary, an enzyme such as protease or a surfactant such as sodium lauryl sulfate (SDS) is used to solubilize the bacterial cells, whereby a water-soluble fraction containing a predetermined modified protein glutaminase can be obtained. It is also possible to secrete the expressed modified protein glutaminase into the culture solution by selecting an appropriate expression cassette or expression vector and host.

The thus-obtained water-soluble fraction containing the modified protein glutaminase may be directly subjected to purification treatment, or may be subjected to purification treatment after the modified protein glutaminase in the water-soluble fraction is concentrated. The concentration can be performed by, for example, concentration under reduced pressure, membrane concentration, salting-out treatment, or fractional precipitation with hydrophilic organic solvents (for example, methanol, ethanol, and acetone).

The purification treatment of the modified protein glutaminase can be performed by, for example, appropriately combining methods such as gel filtration, adsorption chromatography, ion-exchange chromatography, and affinity chromatography. The purified modified protein glutaminase may be powderized by lyophilization, vacuum drying, spray drying, or the like if necessary.

### 6. Enzyme agent

The modified protein glutaminase can be provided in the form of an enzyme agent. Accordingly, the present invention also provides an enzyme agent containing the modified protein glutaminase described in "1. Modified protein glutaminase" above as an active ingredient.

The content of the modified protein glutaminase in the enzyme agent of the present invention is not limited, and the lower limit of the content is, for example, 1 U/g or more, preferably 10 U/g or more, more preferably 50 U/g or more, still more preferably 100 U/g or more, and particularly preferably 200 U/g or more. The upper limit of the content is, for example, 10,000 U/g or less, preferably 5,000 U/g or less, more preferably 2,000 U/g or less, still more preferably 1,000 U/g or less, and particularly preferably 800 U/g or less.

The enzyme agent of the present invention may, or is not required to, contain, in addition to the modified protein glutaminase, other components to the extent that the effect of the present invention is not affected. Examples of the other component include other enzymes other than the modified protein glutaminase, additives, and culture residues generated by the above-described production method.

Examples of the other enzyme include amylase (α-amylase, β-amylase, glucoamylase), glucosidase (α-glucosidase, β-glucosidase), galactosidase (α-galactosidase, β-galactosidase), protease (acidic protease, neutral protease, alkaline protease), peptidase (leucine peptidase, aminopeptidase), lipase, esterase, cellulase, phosphatase (acid phosphatase, alkaline phosphatase), nuclease, deaminase, oxidase, dehydrogenase, glutaminase, pectinase, catalase, dextranase, transglutaminase, protein deamidase (except for the above-described modified protein glutaminase), and pullulanase. The other enzymes may be contained alone, or in combination of two or more thereof.

Examples of the additive include an excipient, a buffer, a suspending agent, a stabilizer, a preservative, an antiseptic agent, and physiological saline. Examples of the excipient include starch, dextrin, maltose, trehalose, lactose, D-glucose, sorbitol, D-mannitol, sucrose, and glycerol. Examples of the buffer include phosphate, citrate, and acetate. Examples of the stabilizer include propylene glycol and ascorbic acid. Examples of the preservative include phenol, benzalkonium chloride, benzyl alcohol, chlorobutanol, and methylparaben. Examples of the antiseptic agent include ethanol, benzalkonium chloride, paraoxybenzoic acid, and chlorobutanol. These additives may be contained alone, or in combination of two or more thereof.

Examples of the culture residue include a component derived from a culture medium, contaminating protein, and a bacterial cell component.

The form of the enzyme agent of the present invention is not particularly limited, and examples thereof include a liquid form and a solid form (powder, granules, and the like). The enzyme agent in the above-described form can be prepared by a generally known method.

### 7. Modifier for protein material

The modified protein glutaminase can be used for known applications of protein glutaminase. For example, the modified protein glutaminase can be used for the purpose of modifying a protein material. Accordingly, the present invention also provides a modifier for a protein material which contains a modified protein glutaminase.

The specific aspect of the modification of a protein material is not limited as long as it is a change in property of protein which is caused by generation of a carboxyl group due to deamidation of the γ-amide group and the β-amide group of the glutamine residue and the asparagine residue of the protein. Specifically, examples of the modification of a protein material include enhancement of solubility of protein, enhancement of water dispersibility, improvement of emulsifying capacity, and emulsion stability. A specific method for using the modifier for a protein material is as described in "8. Method for producing modified protein material" below.

### 8. Method for producing modified protein material

As described above, the modified protein glutaminase can be used for the purpose of modifying a protein material. Accordingly, the present invention also provides a method for producing a modified protein material, including the step of applying the modified protein glutaminase to a protein material.

In the production method of the present invention, a mixture containing a protein material and a modified protein glutaminase is placed under conditions for application of the modified protein glutaminase to make a protein modifying reaction proceed.

The protein material is not particularly limited as long as it contains protein. The protein material may either one for edible use or one for non-food use. The edible protein material can be used as a food/drink or as a material for producing a food/drink. The non-food protein material can be used as a material for protein experiments, a medical material, a fiber material, a cosmetic material, or the like.

Specific examples of the protein material include a protein source itself, and a preparation obtained from a protein source by performing treatment for increasing the protein content using a known method, which are appropriately selected by those skilled in the art. Examples of the edible protein material include a preparation obtained from a food product containing plant protein as a plant protein material; and a preparation prepared from a food product containing animal protein as an animal protein material. Examples of the edible plant protein include bean protein such as soybean protein, broad bean protein, pea protein, chickpea bean protein, green bean protein and lupin bean protein; grain protein such as wheat protein, rye protein, oat protein and corn protein; and seed protein from canary seed, linseed, almond, cashew nut, hazelnut, pecan nut, macadamia nut, pistachio, walnut, brazil nut, peanut, coconut, hemp seed (industrial hemp), pili, chestnut, sesame, pine nut, and the like. Examples of the edible animal protein include protein from livestock meat, fish meat, egg, and milk. Examples of the non-food protein material include albumin and globulin derived from a biological sample such as egg white or serum; and silk, and wool.

The content of the protein in the protein material is not limited, and is, for example, 30 wt% or more, 40 wt% or more, or 50 wt% or more, preferably 60 wt% or more, more preferably 70 wt% or more, and still more preferably 80 wt% or more. The upper limit of the content of the protein in the protein material is not limited, but is, for example, 95 wt% or less, 90 wt% or less, 85 wt% or less, 80 wt% or less, 70 wt% or less, or 60 wt% or less.

Examples of the content of the protein material in the mixture include an amount such that the concentration of protein contained in the protein material in the mixture is, for example, 0.1 wt% or more, or 0.3 wt% or more, preferably 0.7 wt% or more, and more preferably 1.4 wt% or more. The upper limit of the concentration of protein contained in the protein material in the mixture is not limited, but is, for example, 80 wt% or less, 60 wt% or less, 40 wt% or less, 30 wt% or less, 20 wt% or less, 15 wt% or less, 10 wt% or less, 8 wt% or less, 5 wt% or less, 3 wt% or less, or 2 wt% or less.

The amount of the modified protein glutaminase used is not limited, and the amount of the modified protein glutaminase used per gram of protein contained in the protein material is, for example, 0.1 U or more, preferably 0.5 U or more, more preferably 1 U or more, still more preferably 2 U or more, even more preferably 3.5 U or more, furthermore preferably 4.5 U or more. The upper limit of the amount of the modified protein glutaminase used per gram of protein contained in the protein material is not limited, and is, for example, 45 U or less, 35 U or less, 20 U or less, 10 U or less, 8 U or less, or 5.5 U or less.

The amount of the modified protein glutaminase used per gram of the protein material is, for example, 0.01 U or more, 0.05 U or more, or 0.1 U or more, preferably 0.5 U or more, more preferably 1 U or more, still more preferably 2 U or more, even more preferably 3 U or more, and furthermore preferably 4 U or more. The upper limit of the amount of the modified protein glutaminase used per gram of the protein material is not limited, and is, for example, 40 U or less, 30 U or less, 20 U or less, 10 U or less, 7 U or less, or 5 U or less.

The conditions for application of the modified protein glutaminase are appropriately determined on the basis of the optimal temperature and optimal pH for the modified protein glutaminase used.

The temperature condition among the conditions for application of the modified protein glutaminase can be appropriately determined according to the optimal temperature for the modified protein glutaminase, and the like by those skilled in the art. As a specific temperature condition, the temperature is, for example, 40 to 70°C, preferably 48 to 67°C, more preferably 53 to 65°C, and still more preferably 57 to 63°C.

The pH condition among the conditions for application of the modified protein glutaminase can be appropriately determined according to the optimal pH for the modified protein glutaminase, and the like by those skilled in the art. As a specific pH condition, the pH is, for example, 2 to 12, preferably 3 to 10, and more preferably 4 to 9.

The time of applying the modified protein glutaminase is not limited, and may be appropriately determined according to the preparation scale or the like, and is, for example, 1 hour or more, preferably 8 hours or more, more preferably 16 hours or more, and still more preferably 20 hours or more. The upper limit of the range of the time is not limited, and is, for example, 40 hours or less, 30 hours or less, or 25 hours or less.

After completion of the reaction, enzyme deactivation treatment is performed, followed by cooling, and, if necessary, posttreatment is performed to obtain a modified protein material.

### Examples

Hereinafter, the present invention will be described in detail by way of examples, which should not be construed as limiting the present invention.

### Test Example 1

### (1) Preparation of modified protein glutaminase

A sequence (SEQ ID NO: 11) encoding the amino acid sequence (SEQ ID NO: 1) of protein glutaminase derived from C. proteolyticum and introduced into pET21 vector was used as a template, into which various mutations were introduced to prepare the following modified protein glutaminases.

**[Table 1]**

| | |
|---|---|
| Example 1 | C121V mutant of SEQ ID NO: 1 (SEQ ID NO: 3) |
| Example 2 | C121A mutant of SEQ ID NO: 1 (SEQ ID NO: 4) |
| Example 3 | Y142C mutant of SEQ ID NO: 1 (SEQ ID NO: 5) |
| Example 4 | Y142D mutant of SEQ ID NO: 1 (SEQ ID NO: 6) |

Specifically, using the following primers and PrimeSTAR Mutagenesis Basal Kit (Takara), PCR was performed by a conventional method to introduce mutations.
(Primer for C121V mutation)
   Rv: 5'-ATCTGTTACAGGACCGCTTGAAAATAGTGAAGGATC-3' (SEQ ID NO: 19) 
Fw: 5’-
ACAGCATGGAGAAACGCTgttGTTAACACCTCTTGCGGATCTGCATCC-3’ (SEQ
ID NO: 20)
(Primer for C121A mutation)
   Rv: 5'-ATCTGTTACAGGACCGCTTGAAAATAGTGAAGGATC-3' (SEQ ID NO: 21) 
Fw: 5’-
ACAGCATGGAGAAACGCTgcgGTTAACACCTCTTGCGGATCTGCATCC-3’
(SEQ ID NO: 22)
(Primer for Y142C mutation)
   Rv: 5'-CTCTTGCGGATCTGCATCCGTTTCCTCTTATGCT-3' (SEQ ID NO: 23) 
Fw: 5’-
AATACTGCAGGAAATGTTtgtTACAGAAGTCCTAGTAATTCTTACCTGTATGA
CAACAATC-3’ (SEQ ID NO: 24)
(Primer for Y142D mutation)
   Rv: 5'-CTCTTGCGGATCTGCATCCGTTTCCTCTTATGCT-3' (SEQ ID NO: 25)

Fw: 5’-
AATACTGCAGGAAATGTTgatTACAGAAGTCCTAGTAATTCTTACCTGTATGA
CAACAATC-3’ (SEQ ID NO: 26)

With each of the obtained mutated gene products, E. coli BL21 (DE3) was transformed by a conventional method to acquire gene expression vectors. The protein glutaminase sequence introduced into the gene expression vector was confirmed. The transformant was cultured with shaking in LB medium at 37°C for 16 hours. Thereafter, the transformant was transplanted to Terrific Broth, and cultured at 37°C for 4 hours, and IPTG (final concentration: 0.5 mM) was added, followed by shaking culture at 33°C for 20 hours. The bacterial cells were collected from the culture solution, and the bacterial cells were lysed with B-PER (trademark) Bacterial Cell Lysis Reagent (manufactured by Thermo Scientific) by a conventional method, and centrifuged at 15,000 rpm for 10 minutes. The centrifugal supernatant was collected as a crude enzyme solution, trypsin was added to the solution at a final concentration of 50 ug/mL, and the mixture was allowed to stand at 37°C for 1 hour, and then purified by a conventional method using TALON (registered trademark) Spin Columns (manufactured by Takara Bio Inc.), thereby obtaining an enzyme sample.

### (2) Method for measuring protein glutaminase activity

N-Benzyloxycarbonyl-L-glutaminylglycine (Z-Gln-Gly; Peptide Institute, Inc.) was dissolved in a 0.2 mol/L phosphate buffer (pH 6.5) at 30 mmol/L, and the thusprepared solution was used as a substrate solution. To a test tube, 0.1 mL of an enzyme solution whose activity was to be measured was added, and left standing in a constant-temperature water bath at 37 ± 0.5°C for 1 minute. Thereafter, 1 mL of a substrate solution left to stand at 37 ± 0.5°C for 10 minutes in advance was added, and the mixture was immediately mixed. This solution was left standing for 10 minutes to carry out an enzymatic reaction, and 1 mL of a 0.4 mol/L trichloroacetic acid solution was added to stop the enzymatic reaction. A measurement blank was prepared by adding 0.1 mL of an enzyme solution to a test tube, and adding 1 mL of a 0.4 mol/L of a trichloroacetic acid solution and 1 mL of a substrate solution in this order. A color development reaction by Ammonia-Test Wako (FUJIFILM Wako Pure Chemical Corporation) was carried out, and ammonia released by an enzyme reaction for 10 minutes was quantified by the value of absorbance at a wavelength of 630 nm. The ammonia concentration in the reaction solution was determined from a calibration curve showing the relationship between the ammonia concentration and the absorbance (630 nm), which had been prepared using an ammonia standard solution (ammonium chloride). The enzymatic activity of protein glutaminase was calculated from the following equation, where the amount of an enzyme producing ammonia at 1 µmol per minute is defined as one unit (1U). In the equation, the amount of the reaction solution is 2.1, the amount of the enzyme solution is 0.1, and Df is a dilution factor of the enzyme solution. The value "17.03" denotes the molecular weight of ammonia. Enzymatic activity (U/mL) = ammonia concentration in reaction solution (mg/L) × (1/17.03) × (amount of reaction solution/amount of enzyme solution) × (1/10) × Df

### (3) Confirmation of specific activity

The specific activity at 37°C was measured using the obtained samples of the modified protein glutaminases of Examples 1 to 4. The results showed that the specific activity when the specific activity of Chryseobacterium proteolyticum-derived protein glutaminase (wild-type) is defined as 100% was 84% for the enzyme sample of the modified protein glutaminase of Example 1, 93% for the enzyme sample of the modified protein glutaminase of Example 2, 137% for the enzyme sample of the modified protein glutaminase of Example 3, and 90% for the enzyme sample of the modified protein glutaminase of Example 4, and all the samples were confirmed to be equivalent or superior in activity to the wild-type.

### (4) Confirmation of oxidation stability

To a solution in which the amount of protein in the purified enzyme solution was adjusted to 0.1 mg/mL, hydrogen peroxide was added at a final concentration of 0.001 w/w%. The protein glutaminase activity after the treatment at 37°C for 1 hour was measured, and the remaining activity (post-oxidation treatment remaining activity A_{0.001%}) was calculated from the ratio of the protein glutaminase activity after hydrogen peroxide treatment to the protein glutaminase activity without hydrogen peroxide treatment. The ratio of the post-oxidation treatment remaining activity A_{0.001%} of the modified protein glutaminase to the post-oxidation treatment remaining activity A_{0.001%} of the wild-type protein glutaminase (ratio to wild type) was calculated. Further, the post-oxidation treatment remaining activity B_{0.0025%} and the ratio to wild type were similarly calculated except that the amount of hydrogen peroxide added was changed to a final concentration of 0.0025 w/w%. The results are shown in the following table.

**[Table 2]**

| | | Specific activity (ratio to activity of wild type which is defined as 100%) | 0.001% Hydrogen peroxide treatment | | 0.0025% Hydrogen peroxide treatment | |
|---|---|---|---|---|---|---|
| | | | Post-oxidation treatment activity A_{0.001%} | Ratio to wild type | Post-oxidation treatment activity B_{0.0025%} | Ratio to wild type |
| Reference Example 1 | Wild type | 100% | 9% | 1 | 1% | 1 |
| Example 1 | C121V mutant | 84% | 56% | 5.9 | 23% | 26.1 |
| Example 2 | C121A mutant | 93% | 41% | 4.4 | 7% | 7.9 |
| Example 3 | Y142C mutant | 137% | 58% | 6.2 | 9% | 10.6 |
| Example 4 | Y142D mutant | 90% | 47% | 5 | 20% | 22.8 |

As shown in the table above, the remaining activity after hydrogen peroxide treatment was remarkably improved in the modified protein glutaminases of Examples 1 to 4.

### Test Example 2

Using a random mutation introduction kit (Gene Morph II Random Mutagenesis kit, Diversify PCR Random Mutagenesis Kit, manufactured by Agilent Technologies, Inc.), a random mutation was introduced into the sequence (SEQ ID NO: 11) encoding the amino acid sequence of C. proteolyticum-derived protein glutaminase (SEQ ID NO: 1) according to the instructions for the kit.

The gene after introduction of the mutation was inserted into a pET21a vector using In-Fusion (registered trademark) HD Cloning Kit (manufactured by Clontech Laboratories, Inc.). E. coli BL21 (DE3) was transformed by a conventional method, and the obtained transformant was cultured with shaking in LB medium at 37°C for 16 hours. Thereafter, the transformant was transplanted to Terrific Broth, and cultured at 37°C for 4 hours, and IPTG (final concentration: 0.5 mM) was added, followed by shaking culture at 33°C for 20 hours. The bacterial cells were collected from the culture solution, and the bacterial cells were lysed with B-PER (trademark) Bacterial Cell Lysis Reagent (manufactured by Thermo Scientific) by a conventional method, and centrifuged at 15,000 rpm for 10 minutes. The centrifugal supernatant was collected as a crude enzyme solution, trypsin was added to the solution at a final concentration of 50 ug/mL, and the mixture was allowed to stand at 37°C for 1 hour, and then purified by a conventional method using TALON (registered trademark) Spin Columns (manufactured by Takara Bio Inc.), thereby obtaining an enzyme sample.

The specific activity and oxidation stability of the obtained enzyme sample were evaluated by the method of Test Example 1. For enzyme samples whose oxidation stability was improved, the gene sequence was confirmed, and the mutation was identified. The results are shown in the following table.

**[Table 3]**

| | | Specific activity (ratio to activity of wild type which is defined as 100%) | 0.0025% Hydrogen peroxide treatment | |
|---|---|---|---|---|
| | | | Post-oxidation treatment activity B_{0.0025%} | Ratio to wild type |
| Reference Example 1 | Wild type | 100% | 2% | 1 |
| Example 5 | R361 mutant | 75% | 52% | 25 |
| Example 6 | S30T/T113K mutant | 74% | 23% | 11 |
| Example 7 | I157F mutant | 74% | 25% | 12 |
| Example 8 | L156P mutant | 63% | 52% | 26 |
| Example 9 | S26C/Y43F/C183G mutant | 28% | 79% | 38 |
| Example 10 | I33V mutant | 69% | 66% | 32 |
| Example 11 | L169F mutant | 69% | 32% | 15 |
| Example 12 | S 182R mutant | 76% | 56% | 28 |
| Example 13 | T731 mutant | 31% | 49% | 24 |
| Example 14 | S26Y mutant | 63% | 87% | 42 |
| Example 15 | F35S mutant | 81% | 38% | 18 |
| Example 16 | S22Y/S170T mutant | 111% | 88% | 43 |

As shown in the table above, the remaining activity after hydrogen peroxide treatment was remarkably improved in the modified protein glutaminases of Examples 5 to 16.

### Sequence Listing Free Text

SEQ ID NOS: 19 to 26 correspond to primers.

## Claims

1. A modified protein glutaminase comprising one of the following polypeptides (I) to (III):
(I) a polypeptide consisting of an amino acid sequence obtained by introducing at least one of
(A) a substitution of the amino acid residue at position 121 with a valine residue or an alanine residue,
(B) a substitution of the amino acid residue at position 142 with a cysteine residue or an aspartic acid residue,
(C) a substitution of the amino acid residue at position 22 with a tyrosine residue,
(D) a substitution of the amino acid residue at position 26 with a tyrosine residue or a cysteine residue,
(E) a substitution of the amino acid residue at position 30 with a threonine residue,
(F) a substitution of the amino acid residue at position 33 with a valine residue,
(G) a substitution of the amino acid residue at position 35 with a serine residue,
(H) a substitution of the amino acid residue at position 36 with an isoleucine residue,
(I) a substitution of the amino acid residue at position 43 with a phenylalanine residue,
(J) a substitution of the amino acid residue at position 73 with an isoleucine residue,
(K) a substitution of the amino acid residue at position 113 with a lysine residue,
(L) a substitution of the amino acid residue at position 156 with a proline residue,
(M) a substitution of the amino acid residue at position 157 with a phenylalanine residue,
(N) a substitution of the amino acid residue at position 169 with a phenylalanine residue,
(O) a substitution of the amino acid residue at position 170 with a threonine residue,
(P) a substitution of the amino acid residue at position 182 with an arginine residue, and
(Q) a substitution of the amino acid residue at position 183 with a glycine residue,
into the amino acid sequence set forth as SEQ ID NO: 1;
(II) a polypeptide in which one or several amino acid residues other than the substituted amino acid residues are substituted, added, inserted or deleted in the amino acid sequence in which at least one of the substitutions (A) to (Q) is introduced, and the remaining activity of protein glutaminase after hydrogen peroxide treatment is improved as compared to the remaining activity of the polypeptide consisting of the amino acid sequence set forth as SEQ ID NO: 1; and
(III) a polypeptide in which the sequence identity of regions that do not include the substituted amino acid sequences is 70% or more in the amino acid sequence in which at least one of the substitutions (A) to (Q) is introduced, and the remaining activity of protein glutaminase after hydrogen peroxide treatment is improved as compared to the remaining activity of the polypeptide consisting of the amino acid sequence set forth as SEQ ID NO: 1.

2. A DNA encoding the modified protein glutaminase according to claim 1.

3. An expression cassette or a recombinant vector comprising the DNA according to claim 2.

4. A transformant obtained by transforming a host using the expression cassette or recombinant vector according to claim 3.

5. A method for producing a modified protein glutaminase, comprising the step of culturing the transformant according to claim 4.

6. An enzyme agent comprising the modified protein glutaminase according to claim 1.

7. A modifier for a protein material, comprising the modified protein glutaminase according to claim 1.

8. A method for producing a modified protein material, comprising the step of applying the modified protein glutaminase according to claim 1 to a protein material.
